# EUROPEAN PATENT SPECIFICATION

(11) **EP 2 800 589 B1**
(45) Date of publication and mention of the grant of the patent: **19.10.2016**
(21) Application number: 12815883.9
(22) Date of filing: 13.12.2012
(51) Int. Cl.: A61L 31/02, A61L 31/14, A61N 1/05

(54) **POROUS DEALLOYED ELECTRODES**
PORÖSE ENTLEGIERTE ELEKTRODEN
ÉLECTRODES DÉSALLIÉES POREUSES

(30) Priority: 04.01.2012 US 201261582991 P
(43) Date of publication of application: 12.11.2014
(73) Proprietor: Advanced Bionics AG, 8712 Stäfa (CH)
(72) Inventor: LOTFI, Atoosa, Valencia, California 91354 (US); BEERLING, Timothy, San Francisco, California 94103 (US)
(74) Representative: Schwan Schorer & Partner mbB
(86) International application number: PCT/US2012/069400
(87) International publication number: WO 2013/103489

(56) References cited:
- WO-A1-2011/127395
- US-A1- 2006 121 080
- Dylan Vicente Pugh: "Nanoporous platinum", , 9 May 2003 (2003-05-09), XP055056163, Virginia Polytechnic Institute and State University Retrieved from the Internet: URL:http://scholar.lib.vt.edu/theses/avail able/etd-04252003-104756/unrestricted/Diss ertationPugh.pdf [retrieved on 2013-03-12]
- David Giamov: "Surface modification of biomaterials for use as neural stimulators", , 1 January 1997 (1997-01-01), XP55056166, University of Toronto Retrieved from the Internet: URL:https://tspace.library.utoronto.ca/han dle/1807/11586 [retrieved on 2013-03-12]

## Description

The present description generally relates to neural prosthetics. The invention is set/out in the appended claims.

### BACKGROUND

Neural prosthetics can be used for electrical stimulation of neural tissue and sensing neural signals from the central and peripheral nervous systems. These neural prosthetics include one or more electrodes that make electrical contact with neural tissue. The charge transfer characteristics of the electrodes define how efficiently the electrodes can transfer or receive electrical signals from the neural tissue.
The thesis "Nanoporous platinum" by D.V. Pugh, Virginia Polytechnic Institute and State University, 2003, relates to a study on electrodes for biomedical sensors made of dealloyed copper platinum porous structures, with the production of porous platinum with pore sizes ranging from 2 to 200 nm having been demonstrated (see Abstract). In particular, it relates to cardiac pacing leads comprising porous pacing tips.
The thesis "Surface modification of biomaterials for use as neural stimulators" by D. Gimaov, University of Toronto, 1997, relates to a surface modification process of biomaterials for use as neural stimulators, wherein a Ti-Al-V alloy is enriched with iridium by ion-implantation and selective dealloying.

### BRIEF DESCRIPTION OF THE DRAWINGS

The accompanying drawings illustrate various embodiments of the principles described herein and are a part of the specification.
Fig. 1A is a cross sectional diagram of an electrode and target tissue with a corresponding electrical schematic, according to one example of principles described herein.
Fig. 1B is a cross sectional diagram of the electrode and target tissue with an accumulation of tissue over the electrode, according to one example of principles described herein.
Figs. 2A-2C are cross sectional views that illustrate dealloying of an electrode, according to one example of principles described herein.
Fig. 2D is a picture produced by a scanning electron microscope of a dealloyed nanoporous surface, according to one example of principles described herein.
Fig. 3 is a cross sectional view of a dealloyed nanoporous electrode that resists accumulation of biological tissue on the electrode, according to one example of principles described herein.
Figs. 4A and 4B are cross sectional diagrams that describe dealloying of a bilayer electrode, according to one example of principles described herein.
Figs. 4C and 4D are partial cross sectional and cross sectional diagrams showing the construction and dealloying of a trilayer structure to form a nanoporous dealloyed electrode, according to one example of principles described herein.
Fig. 5A is a diagram of a cochlear electrode array being inserted into a cochlea, according to one example of principles described herein.
Fig. 5B is a top view of a patterned sheet of flexible conductive material that is attached to an underlying support component, according to one example of principles described herein.
Fig. 5C is a perspective view of a winged construct folded to form an electrode, according to one example of principles described herein.
Fig. 5D is a cross sectional view of the folded winged construct and signal wires embedded in a flexible body, according to one example of principles described herein.
Figs. 5E-5G are cross sectional views of removal of the support component and dealloying of the surface of the electrode, according to one example of principles described herein.
Figs. 5H-5L are cross sectional views of various stages in an illustrative process for forming a nanoporous dealloyed electrode, according to one example of principles described herein.
Fig. 6 is a flowchart diagram of an illustrative method for forming a nanoporous dealloyed electrode, according to one example of principles described herein.
Fig. 7 is a flowchart diagram of an illustrative method for forming an electrode array with nanoporous dealloyed electrodes, according to one example of principles described herein.

Throughout the drawings, identical reference numbers designate similar, but not necessarily identical, elements.

### DETAILED DESCRIPTION

The Invention relates to a cochlear implant device as defined in claim 1 and method for forming an implantable cochlear electrode as defined in claim 6. The embodiments, aspects or examples of the present descrption that do not fall within the scope of said claims are provided for illustrative purposes only and do not form part of the invention.

Neural prosthetics use electrodes to electrically stimulate neural tissue and sense electrical signals from neural tissue. The charge transfer between the electrodes and neural tissue is in part determined by the electrochemistry of the electrode/tissue interface. To avoid energy losses and distortions of electrical signals, the electrical impedance of the interface should be very small in the desired stimulation/recording frequency range. The electrical impedance of the interface is the combination of the impedance of the electrode and the impedance of biological environment surrounding the electrode.

Transfer of charge within the neural prosthetic device and its electrodes is carried out by flow of electrons. Within the biological environment however, transfer of charge is carried out by flow of ionic current between two or more electrodes. Therefore, to mediate the transition from electron flow in the electrode to ion flow in the biological environment and to minimize the impedance of the interface, the material, size, shape and topography of the electrodes need to be carefully selected.

However, changes in the biological environment surrounding the electrodes, such as changes in protein constituent of the electrolyte and the fibrous tissue encapsulation of the electrodes, can significantly increase the impedance of the interface. Increased impedance negatively affects the efficiency of stimulation and power consumption of the device. The interaction of the electrode surface with the biological environment can play a significant role in modulating the cellular events at the interface. These cellular events include protein adsorption, cell adhesion, and proliferation. Therefore, any intervention in reducing the impedance of the electrodes by changing the chemical and/or physical characteristics of the electrode can be done in a way that takes into consideration the influences these changes will have on the cellular events at the interface.

As used in the specification and appended claims, the term "support component" is used to describe a component that serves a temporary structural function and is then partly or completely removed after serving that function. The removable of the support component may occur through a variety of processes including chemical etching.

The absolute size and comparative sizes of objects illustrated in the figures is not necessarily to scale. The sizes of the objects have been illustrated to convey the principles described herein.

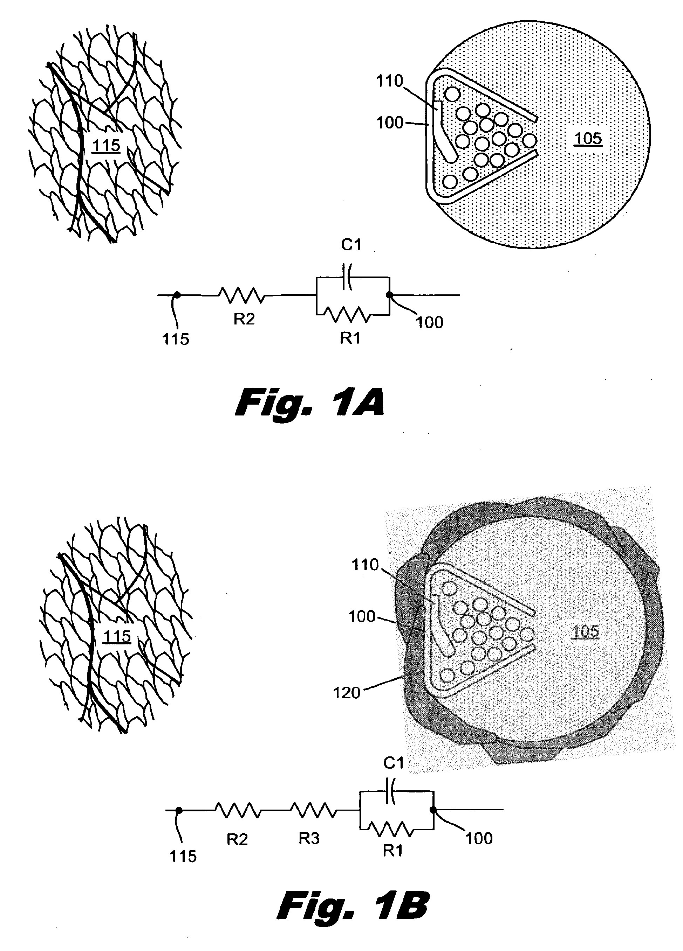

Fig. 1A is a diagram of an electrode (100) implanted in proximity to target biological tissue (115). The electrode (100) is electrically connected to a signal wire (110) and supported by an implant body (105). Ideally, electrical signals pass down the signal wire (110) to the electrode (100) and from the electrode (100) to the target biological tissue (115). For purposes of illustration, the electrode (100) is illustrated as being separated from the target biological tissue (115). For example, in a cochlear implant the electrode (100) may be separated from the target auditory nerve endings by the cochlear fluid and a wall of a cochlear duct. However, the principles described below apply to electrodes that are in direct contact with the target tissue as well as configurations that have intervening fluids and/or tissue between the electrode and target tissue.

A simplified electrical schematic of the interface between the electrode (100) and the target biological tissue (115) is shown below the cross sectional diagram. In the schematic, a first black circle on the left represents the electrode (100) and a second black circle on the right represents the target biological tissue (115). A first resistor R1 and a capacitor C1 represent the impedance at the surface of the electrode (100). The surface resistance R1 and capacitance C1 can be dependent on a number of factors, including the exposed geometric surface area (GSA), the roughness of the electrode and the material that makes up the surface of electrode. For example, increasing the size of the electrode will increase its exposed surface area and lower impedance.

The electrical resistance of the biological environment between the electrode (100) and the target biological tissue (115) is shown by a second resistance R2. The second resistance R2 can be dependent on a number of factors including the concentrations of ions and proteins in fluids, the type of intervening tissues, and the distance between the electrode and the target tissue.

Fig. 1A represents the electrode (100) when it was first implanted in the biological tissue. Fig. 1B shows a simplified diagram of the electrode (100) after being implanted in the biological tissue for a time. The initial response to the surgical implantation of the electrode is nonspecific adsorption of the proteins to the surface of the implant. Nonspecific adsorption of the proteins to the surface of the implant signals the presence of a foreign entity to the immune system. This alters the normal healing process, aggravates the immune response and causes a foreign body reaction that results in encapsulation of the foreign entity material with a fibrous layer.

Fig. 1B shows the electrode encapsulated by a fibrous layer (120). This fibrous layer (120) has a significant electrical resistance that impedes electrical stimulation of the target tissue (115) by the electrode (100). The electrical resistance of the fibrous layer (120) is shown as resistance R3. In some designs, the resistance R3 is so significant that it overwhelms improvements made in the design of the electrode (100) (geometry, shape, material) for the purpose of reducing the impedance of the interface. As a result of the growth of the fibrous layer (120), impedance of the interface increases, with a corresponding increase in power consumption of the implanted device. Consequently, it is desirable to simultaneously improve the charge transfer characteristics of the electrode surface while preventing fibrosis.

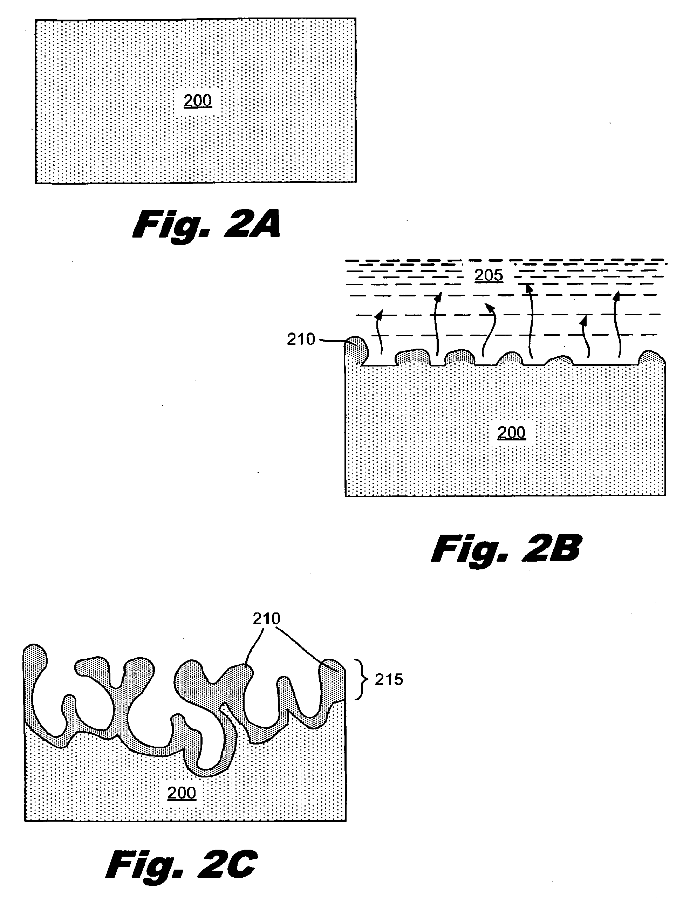

Figs. 2A-2C show an illustrative dealloying technique that simultaneously increases the electrochemical surface area (ESA) of an electrode in contact with the biological fluid (electrolyte) and, in certain configurations, hampers fibrous tissue formation. Additionally, the dealloying technique may change the surface composition of the electrode for increased charge transfer and greater chemical stability.

Dealloying Is selective dissolution of a more electrochemically active element in an alloy. This process results in the formation of a porous structure almost entirely composed of the more noble metal. Sizes of the porosities can be tailored to nanoscale, resulting into nanoporous dealloyed structure. Dealloying the surface of an electrode lowers the impedance at the electrode/tissue interface. This results in lower power consumption, possibilities for array miniaturization (smaller size electrodes can be used), improved selectivity and spatial resolution (larger number of small electrodes can be used for a given array size). The nanoporous dealloyed surface significantly affects both protein adsorption and cellular response. Dealloying changes the surface topography of the electrode and increases the electrochemical surface area while hampering cell adhesion and proliferation. Additionally, the dealloying is a flexible process that is readily scalable and allows for selective surface modification. The dealloying process can be readily integrated into the implanted device process line with minimal increases in complexity or cost of manufacturing.

Fig. 2A shows a cross section of an illustrative alloy (200) prior to dealloying. The alloy may include a number of metals, including, but not limited to platinum, gold, silver, copper, iridium, tungsten, tantalum, palladium, rhodium, ruthenium, iron and other metals. For example, the alloy may comprise platinum alloyed with gold, silver, copper, iridium, tungsten, palladium, rhodium, ruthenium, or other material. The alloy may be a binary, ternary, or other alloy. The alloy may be substantially homogeneous or may contain various structured inhomogeneous structures.

Fig. 2B shows a beginning stage of the dealloying process where the less noble metal in the alloy goes into solution due to chemical or electrochemical action and the more noble metal consolidates into islands (210). The less noble metal continues diffuse into the solution (205) as shown by the upwardly extending arrows. The consolidation of the more noble metal exposes additional surface area that is subsequently dealloyed.

Fig. 2C shows a dealloyed surface (215) that has become nanoporous. The islands (210) have become higher aspect ratio structures separated by significant voids. This dealloyed surface (215) can be in direct contact with the biological environment or can serve as a substrate to be coated by another material of choice to improve functionality.

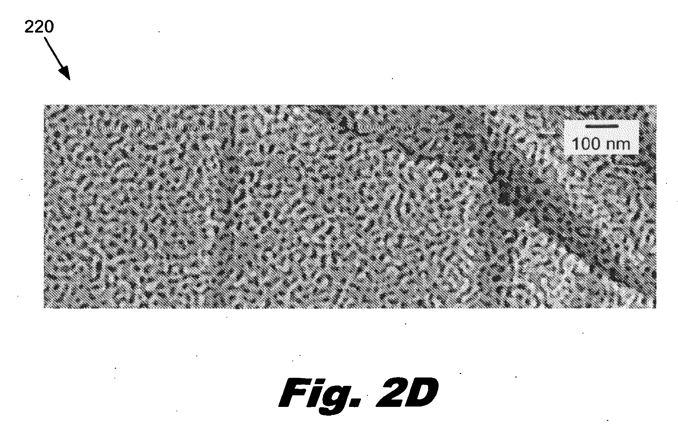

Fig. 2D shows a scanning electron microscope image (220) of a dealloyed surface. The image shows the significant porosity and nanometer scale structures that are achievable using dealloying. As discussed above, the dealloyed surfaces modulate the immune response and the foreign body reactions.

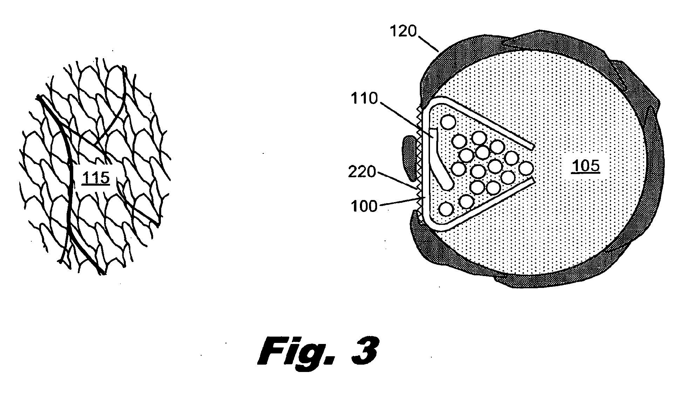

Fig. 3 shows an illustrative electrode (125) with a dealloyed surface (220) after implantation. The increased surface area of the dealloyed surface (220) significantly increases the charge transfer to the target tissue (115). Additionally, the dealloying process has created nanoscale features on the dealloyed surface (220) that resist cellular adhesion. Fibroblasts (120) have adhered to the body (105). However, a substantial number of fibroblasts have not adhered to the surface of the electrode (125). Consequently, the electrical impedance between the electrode (125) and the target tissue (115) does not significantly increase. This provides a number of benefits including longer battery life and possibility for introducing new system architectures (e.g. fully implantable systems).

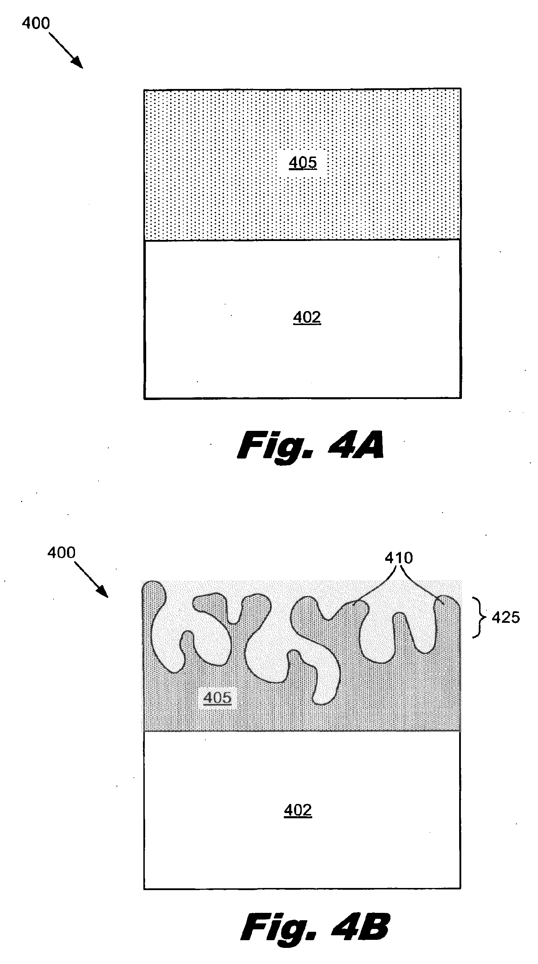

Figs. 2A-2C show an illustrative dealloying process that is performed on an electrode with a homogeneous cross section. However, the dealloying process could be applied to electrodes with a variety of cross sections. Figs. 4A-4C describe dealloying applied to an electrode with a layered cross section. Fig. 4A shows an electrode (400) that initially includes a substrate (402) and an overcoat (405). The overcoat (405) could be deposited on the substrate (402) in a number of ways. For example, the overcoat (405) could be deposited using physical vapor deposition, chemical vapor deposition, cladding (such as laser cladding), sputtering, or electrochemical plating or other deposition methods. The overcoat (405) could include some of the same metals used in the substrate or could use entirely different metals. The thickness of the overcoat (405) can be adjusted to the specific design requirements and can be significantly thinner or thicker than the substrate. In one implementation, the substrate may be formed from a platinum or platinum alloy and the overcoat could be formed from a nickel/iridium or copper/iridium alloy. The copper or nickel in the iridium alloy can be removed via the dealloying process to produce a nanoporous iridium surface.

The pore size, structure spacing/height, and morphology of the nanostructures can have significant effect on the adhesion of fibroblasts and other connective tissue to the electrode surface.

The size, spacing, and porosity of the nanostructure created by the dealloying process can be controlled in a number of ways. For example, the temperature of the dealloying solution, the concentration of etchants in the dealloying solution, the applied voltage and current density, and other factors can be adjusted to produce the desired porous structure. The kinetics of the dealloying process can be controlled so that nano and micro structures with desired aspect ratio are produced.

Fig. 4B shows dealloying of the overcoat (405) of the layered electrode (400). As discussed above, the dealloying produces concentrations of the more noble metal at the dealloyed surface. In this embodiment, the more noble metal forms a layer (425) that covers the remaining portions of the overcoat. The process is controlled to produce nanoscale islands (410) that are composed primarily of the more noble metal. In other embodiments, the dealloying process is configured to remove substantially all of the less noble metal from the overcoat layer.

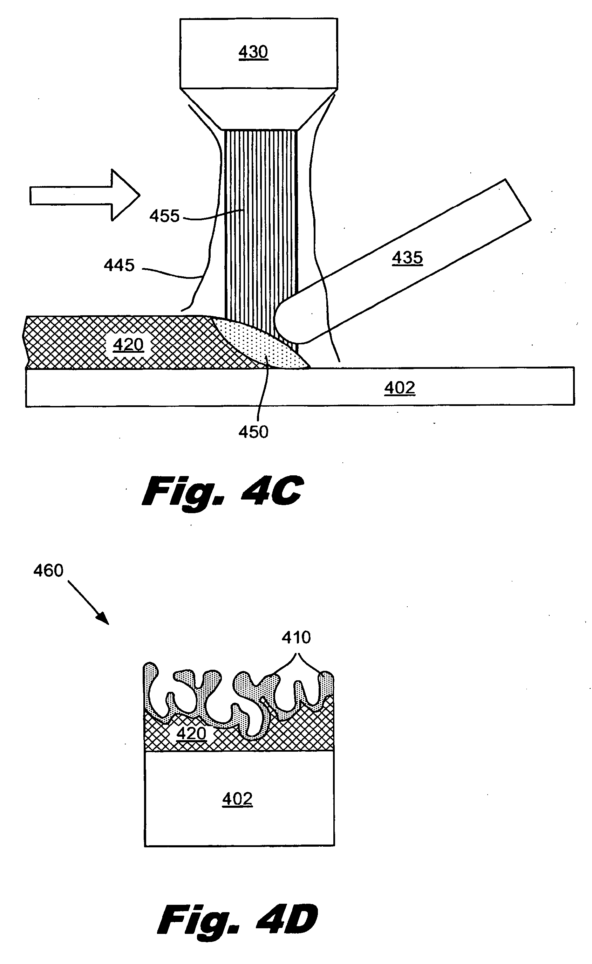

A variety of other processes could be used to form and dealloy the electrode. Fig. 4C shows a laser cladding process that creates electrodes with an initial configuration that has three distinct layers. A substrate (402) serves as a base structure onto which additional material is deposited. The laser beam (455) emanates from a laser head (430) and is surrounded by a shield gas (445). A metal material (435) is introduced from the side into a laser beam (455). For example, the metal material (435) may be a wire, foil, strip, or particles of metal. The laser beam (455) melts the metal material (440) and a portion of the substrate (402) to create a melt pool (450). The melt pool (450) solidifies to form a deposited layer (420). The laser head (430) and the metal insertion mechanism are scanned across the substrate (402) as shown by the arrow. For example, substrate (402) may be platinum, deposited layer (420) may be an iridium alloy and the remelt zone is a combination of platinum from the substrate (402) and deposited iridium alloy. Laser cladding has a number of advantages including compatibility with a wide range of metals or combination of metals and superior bonding with the substrate.

Fig. 4D shows a dealloyed trilayer electrode (460) formed from a laser clad substrate. The dealloyed trilayer electrode (460) includes the substrate (402), remaining portions of the deposited layer (420) and a nanoporous dealloyed layer with a number of islands (410).

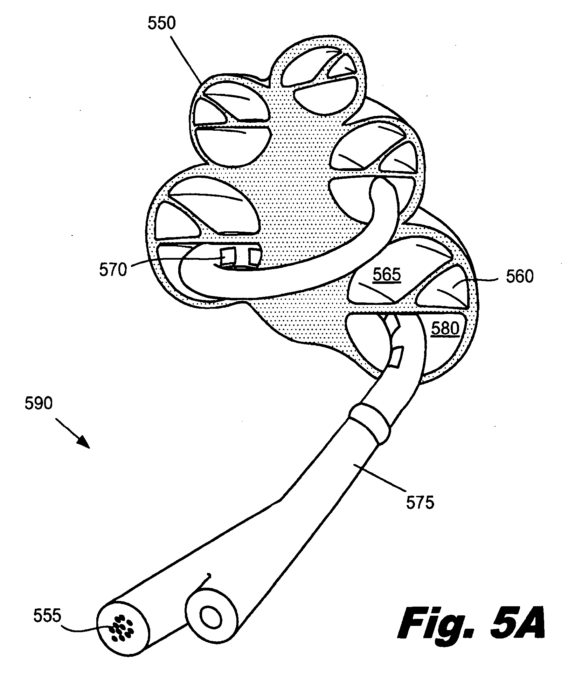

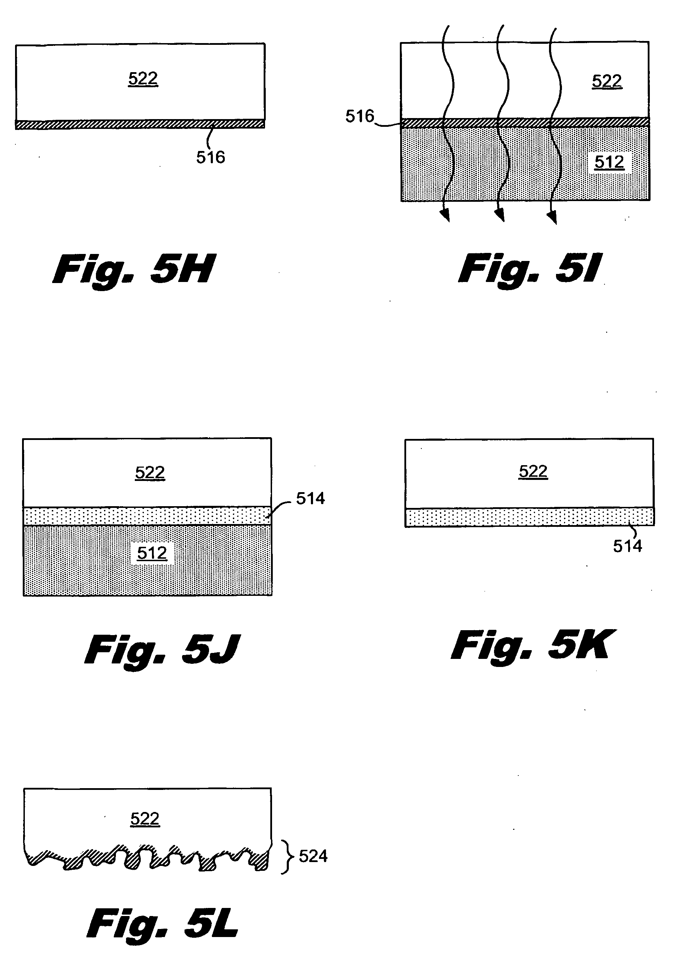

Figs. 5A-5G describe the creation of a cochlear electrode array using dealloying techniques. Fig. 5A is a partially cut away perspective view of a cochlea (550) and shows an illustrative electrode array (595) being inserted into the cochlea (550). The primary structure of the cochlea (550) is a hollow, helically coiled, tubular bone, similar to a nautilus shell. The coiled tube is divided through most of its length into three fluid-filled spaces (scalae) (560, 565, 580). The cochlear lead (590) is inserted into one of the scalae, typically the scala tympani (580), to bring the individual electrodes (570) into close proximity with the tonotopically organized auditory nerves in the cochlea (550).

The illustrative cochlear lead (590) includes a flexible body (575). A number of wires (555) pass through the flexible body (575) to bring electrical signals to the electrodes (570). The electrodes (570) are encased in a flexible body (575) with one surface exposed to biological fluids and tissues. The flexible body (575) may be formed from a variety of biocompatible materials, including, but not limited to medical grade silicone rubber. The flexible body (575) allows the electrode array to bend and conform to the geometry of the cochlea (550).

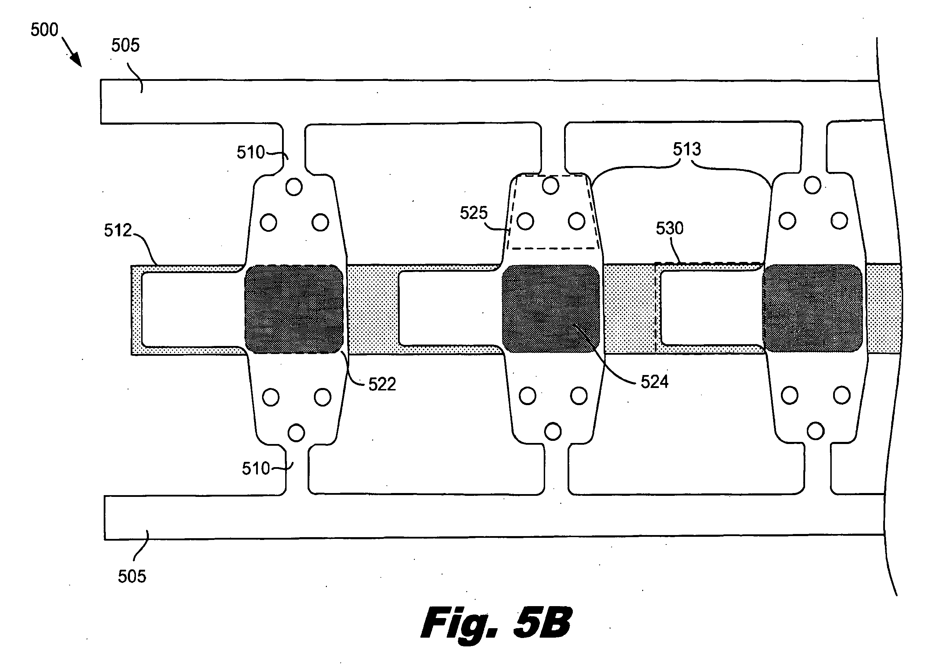

Fig. 5B shows an intermediate step in forming a nanoporous dealloyed cochlear electrode array (595, Fig. 5A). A tethered set (500) of winged tabs (513) have been machined from a flexible electrically conductive material. The manufacture and use of a tethered set of winged tabs to create an electrode array is described in PCT App. Pub. No. WO2011075480, entitled "Cochlear Electrode Array" to Timothy Beerling et al. For example, the conductive material may be between 20 and 50 micron thick platinum or platinum alloy (such as platinum/iridium having up to 20% iridium). Each of the winged tabs (513) is joined to an underlying support component (502). In this embodiment, the winged tabs (513) are resistance welded to the support component (512). The welds (524) cover substantially the pad (522) outlined by the dashed rectangle. The underside of the pad (522) will be exposed to biological tissues. In this example, the pads (522) and welds (524) have a generally rectangular shape. However, the pads (522) and welds (524) could have a variety of other shapes. The welds (524) serve a number of purposes. The welds (524) join the winged tabs (513) to the support component (512). Additionally, the welding process can be controlled so that an alloy layer is formed between the support component (512) and the winged tabs (513). This alloy layer is a combination of a metal or metals contained in the support component and the metal of the pad (522). This alloy layer can later be dealloyed as described above to improve the charge transfer of the exposed surface of the electrode pad (522).

The dashed trapezoid illustrates the wing portions (525) that will be folded up to contain the wires. A second dashed rectangle outlines a flap (530) that will later be folded over a wire and welded to mechanically secure the wire to the electrode. After welding the tethers (510) are cut and the tethers and rails (505) are removed.

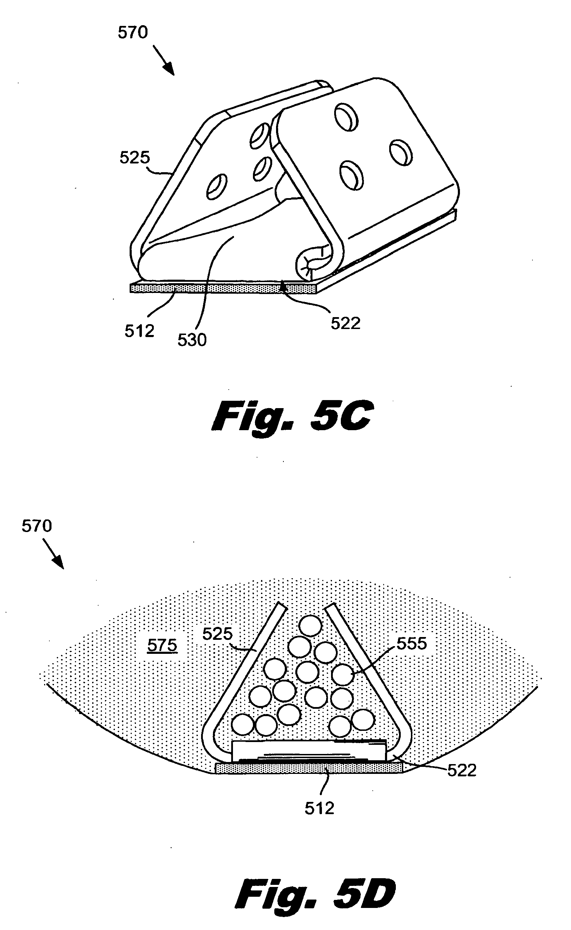

Fig. 5C is a perspective diagram of an illustrative electrode (570) that has been folded to form an integral wire carrier. The overall size of the finished electrode (570) may be on the order of millimeters or less than a millimeter, with feature sizes on the order of tens to hundreds of microns. To form the electrode assembly, the flap (530) of the winged tab is folded over a selected wire and welded to electrically and mechanically secure the wire to the winged tab. For clarity of illustration, the wires are not shown in Fig. 5C. The wings (525) are folded up to secure wires that pass over the electrode to more distal electrodes. A portion of the support component (512) is shown welded to the underside the pad (522).

Fig. 5D is a cross sectional view of the electrode (570) and wires (555) encapsulated in the flexible body (575). The wires (555) are formed into a wire bundle by the wings (525). As discussed above, this wire bundle passes through the entire length of the electrode array (595, Fig. 5A); however, each individual wire within the bundle terminates at the electrode to which it is welded. The support component (512) holds the electrodes (570) in place throughout the molding process and ensures that the bottom surface of the pad (522) is not exposed to the encapsulant. Although the wires (555) are shown as having circular cross sections, electrical conductors with wide range of cross sections could be used.

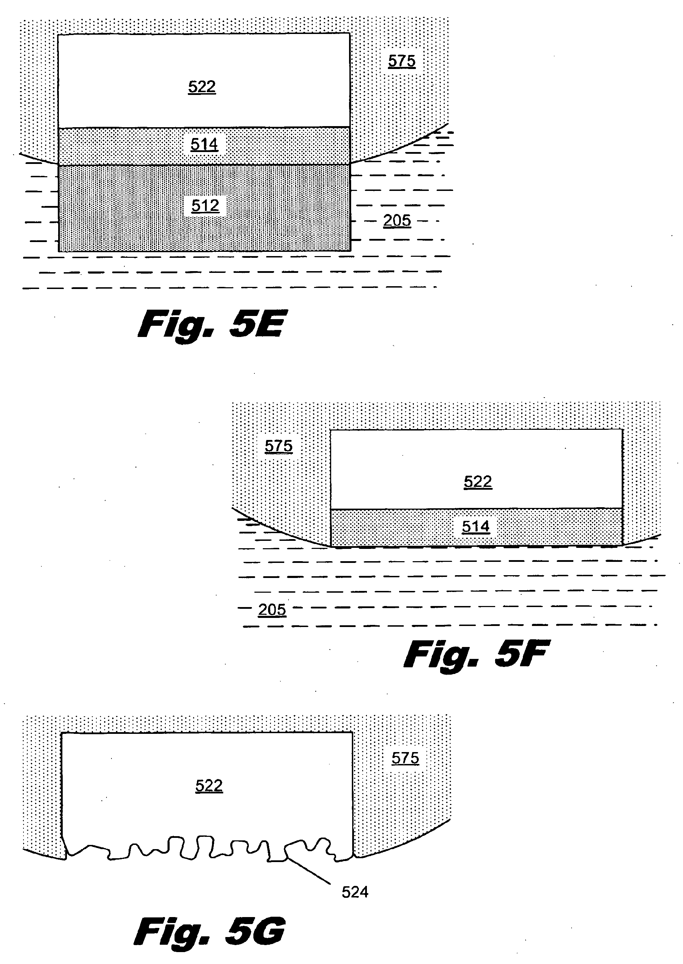

Figs. 5E-5G show steps in an illustrative process for dealloying the pad surface. Fig. 5E shows is a cross sectional view of the pad (522), alloyed layer (514) and support component (512). After the electrodes are molded into the flexible body (575), the support component (512) is no longer needed and can be removed. According to one example, the support component (512) can be removed using a chemical etching solution (205). The support component (512) may be formed from iron, copper or a variety of other materials. In this example, there is no requirement for the support component to be formed from a biocompatible metal or alloy. As discussed above, the pad (522) may be formed from a platinum, platinum alloy, or other appropriate biocompatible metal. The alloy layer (514) is a combination of the metals in the support component (512) and the pad (522) and is formed during the welding process.

Fig. 5F shows the support component (512) removed and the alloy layer (514) exposed. The alloy layer (514) can then be dealloyed. In one example, the process of chemically etching away the support component (512) and dealloying the alloy layer (514) can be performed without interruption and with the same etch solution (205). In some embodiments, one or more parameters involved in the etching process can be adjusted to more effectively form a nanoporous dealloyed surface. For example, the etchant concentration, applied voltage (in the case of anodic dealloying), temperature, or other parameters can be adjusted. Fig. 5G shows the pad (522) encapsulated in the flexible body (575) with a dealloyed surface (524) exposed. Because substantially all of the less noble metal in the alloy layer is removed, the nanostructure is composed of the biocompatible metal that makes up the pad (522).

Figs. 5H-5L show an alternative example of step in a method for creating nanoporous dealloyed electrodes. Fig. 5H shows that the overcoat (516) covers the lower surface of the pad (522). The overcoat (516) could be formed from a variety of metals or metal alloys. For example, the overcoat (516) may have a lower melting point than the metal that makes up the pad (522). In one example, the overcoat (516) could be gold or a gold alloy. In another example, the overcoat (516) could have a substantially higher electrical resistance than the pad (522). This could facilitate localized heating of the overcoat (516) during the welding process. Although the overcoat (516) is illustrated as a single continuous layer, the overcoat could be made up of a number of discrete layers, a structured combination of layers, or unstructured combinations of layers. Additionally, the overcoat does not necessarily consist entirely of metals. The overcoat may also include a number of carbon or non-carbon materials. The overcoat may be deposited onto the support component (512) or onto the pad (522) in any of a number of ways. For example, the overcoat may be deposited using laser cladding, chemical vapor deposition, physical vapor deposition, plating, mechanical adhesion, or other suitable chemical or physical deposition techniques.

Fig. 5I shows the support component (512) brought into contact with the pad (522) so that the overcoat (516) is sandwiched in between the support component (512) and the pad (522). The pad (522) is then welded to the support substrate (512). The wavy lines passing through the layers illustrate the welding process.

During the welding process, an alloy layer (514) is formed between the pad (522) and the support component (512). The alloy layer (514) is formed by diffusion of metal atoms at the interfaces between the overcoat (516) and the pad (522) and the support component (512).

As discussed above, the support component (512) is removed from the alloy layer (514), leaving the alloy layer (514) firmly bonded to the pad (522). This illustrated in Fig. 5K. The alloy layer (514) is then etched as illustrated in Fig. 5L to leave a nanoporous surface (524). A number of post processing steps be performed after the nanoporous surface (524) is formed. These post processing steps may include passivation, oxidization, cleaning, or other appropriate steps.

In one example, the overcoat (516) is iridium and the support component (512) is iron. The welding process forms a platinum iridium alloy layer (514) by combining the iridium with a platinum or platinum alloy pad (522). The etching process removes the iron and dealloys the platinum iridium alloy (514) to produce a nanoporous structure (524) that is rich in iridium. In some embodiments, the iridium nanoporous surface can be post processed. For example, iridium may be activated by exposing the iridium to a number of electrochemical cycles in a water-based electrolyte to develop an "activated" iridium oxide surface. This increases the charge transfer capacity of the iridium. In some embodiments, the dealloying process may already incorporate a water based electrodes and electro-chemical cycles that activate the nanoporous surface. The new techniques and structures described above maximize the charge transfer of the electrode surface while maintaining the manufacturability of the electrode arrays.

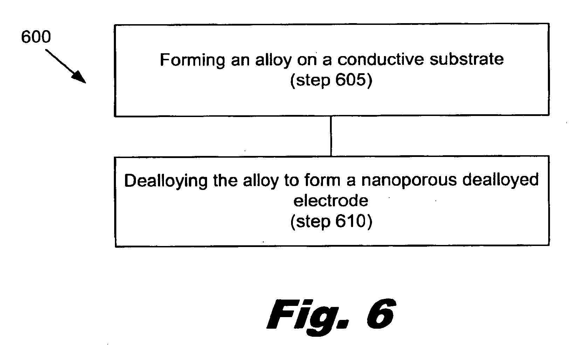

Fig. 6 shows a flowchart (600) of a method for forming a dealloyed electrode. An alloy is formed on a conductive substrate (step 605). The alloy may be formed in a variety of ways. For example, the alloy may be formed through direct deposition of an alloy layer on the conductive substrate using any appropriate deposition technique. For example, the alloy could be formed on the conductive substrate using a chemical or physical deposition technique.

In one example, forming the alloy on the conductive substrate comprises joining a support component to the conductive substrate such that the alloy is formed between the conductive substrate and the support component. In one embodiment, the support component may comprise a coated or uncoated iron strip. For example, the iron strip may be coated with a second metal, such as copper, silver, iridium or gold prior to being joined to the conductive substrate. The support component then is welded to the conductive substrate. A number of intermediate steps can occur that are not illustrated in Fig. 6. For example, the support component may be used to hold the electrode in place during attachment of a conductor to the electrode and during encapsulation in a flexible polymer.

The alloy is dealloyed to form a nanoporous dealloyed electrode (step 610). For example, the support component may be removed through chemical etching to expose the alloy. The alloy is then dealloyed to produce the desired nanostructure. In some implementations, the removal of the support component and the dealloying process may be accomplished using a continuous etching process. For example, the continuous etching process include submerging the device in a etching solution and varying etching parameters, such as solution concentrations, temperature, and electrical potentials (in case of anodic etching) to expeditiously remove the support component and create the desired nanostructure through dealloying. When the device is removed from the bath, the support component has been removed and the electrode surfaces have the desired dealloyed nanostructure. The term "continuous" as applied to an etching process can refer to removing the support structure and dealloying electrode surfaces without removing the device from the etching solution. A continuous etching process may have a number of advantages including shorter process times, reduced cost, lower contamination risks, less waste, and other advantages. In other implementations, the support component may be removed in by a first process and the electrode surfaces dealloyed in a second process.

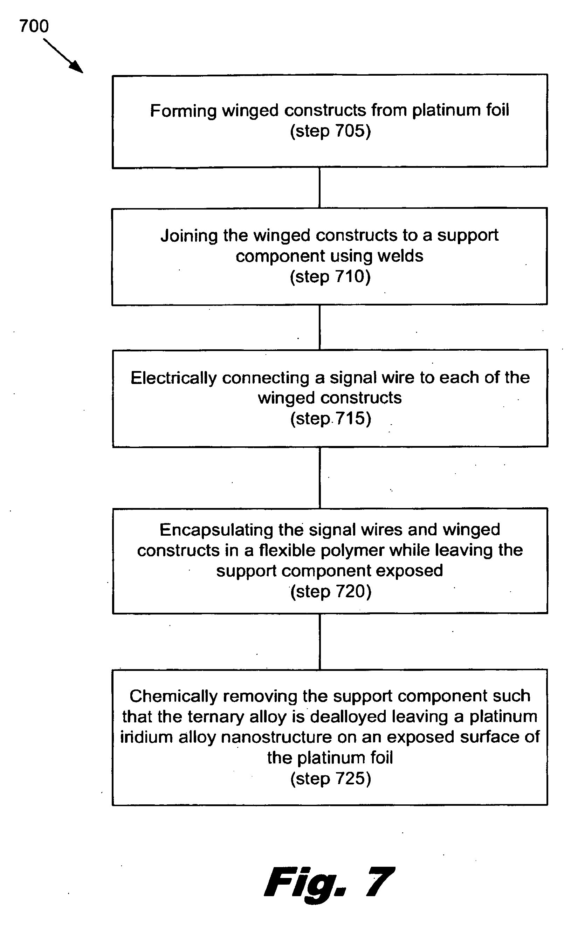

Fig. 7 describes an illustrative method for creating nanoporous dealloyed electrode array that includes forming a plurality of winged constructs from platinum foil (step 705) and joining the winged constructs to a support component using welds (step 710). As discussed above, the support component may have a variety of configurations, compositions, and structures. For example, the support component may include iridium. The welds cover the portion of the winged constructs that will be exposed to biological tissue and form a platinum iridium alloy at an interface between the platinum foil and the support component.

A signal wire is electrically connected to each of the winged constructs (step 715). The signal wires and winged constructs are encapsulated in a flexible polymer while leaving the support component exposed (step 720). The support component is chemically removed such that the ternary alloy of platinum, iridium, and a less noble metal is dealloyed leaving a Pt/ Ir nanostructure on an exposed surface of the platinum foil (step 725). The iridium nanostructure has substantially higher charge transfer characteristics than smooth platinum. The iridium nanostructure is then implanted into biological tissue where it may be used for active stimulation or detection.

The processes and methods described above are illustrative examples and are not limiting. The steps of the processes can be reordered, added, eliminated, or combined. Examples of steps that could be added include activating the iridium nanostructure to create activated iridium oxide and molding steps to encapsulate the wires and portions of the electrodes. Additionally, the steps can be performed in a variety of orders. For example, the wires can be attached before forming the alloy, after forming the alloy but before dealloying, or after dealloying.

In sum, the new techniques and structures described above maximize the charge transfer of the electrode surface over the lifetime of an implant without significantly impacting the manufacturability of the electrode arrays. A dealloyed surface on an electrode could be created from alloyed portions of conductive substrate itself, from an alloyed layer deposited on the substrate or from an alloy generated during joining a support component to the substrate. The dealloying increases the electrochemical surface area of an electrode and may include a metal with a charge transfer capability that is greater than that of the substrate. The dealloyed surface may include nanostructures that inhibit adhesion and growth of biological tissues on the dealloyed surface. By lowering the impedance at the surface of the electrode and ensuring that the impedance remains low throughout the lifetime of the implant, the power consumption of the device can be significantly reduced. Lower power consumption and longer battery life will provide possibility for new system architectures such as fully implantable cochlear systems.

The invention is set out in the following claims.

## Claims

1. A cochlear implant device comprising:
an energy source;
at least one electrode (100, 125, 400, 460, 570) partially embedded in a flexible insulating body (105, 575) and comprising:
an electrically conductive material (220, 522); and
a nanoporous dealloyed material (514) disposed on a portion of the electrically conductive material, the nanoporous dealloyed material being configured to be in electrical contact with the biological tissue, in which the nanoporous dealloyed material has a structure (524) that is configured to resist cellular adhesion and/or fibrous tissue growth and a charge transfer that is higher than that of the electrically conductive material; and
an electrical conductor (110, 555) that conducts current between the energy source and the electrode.

2. The device of claim 1, in which the nanoporous dealloyed material (514) comprises iridium.

3. The device of claim 1, in which only portions of the at least one electrode (100, 125, 400, 460, 570) that are exposed to biological tissue (115) are covered with the nanoporous dealloyed material (514).

4. The device of claim 2, in which the electrically conductive material (220, 522) comprises platinum.

5. The device of claim 1, in which an exposed geometric planar area of the dealloyed material (514) is less than 0.2 square millimeters and the total electrochemical surface area of the dealloyed material is greater than 10 times the geometric planar area.

6. A method for forming the cochlear implant device of claim 1 comprising forming at least one implantable porous dealloyed cochlear electrode (100, 125, 400, 460, 570) comprising:
attaching a signal wire (110, 555) to a conductive substrate (220, 522);
forming an alloy (514) on the conductive substrate; and
dealloying the alloy to form a surface structure (524) that is configured to resist cellular adhesion and/or fibrous tissue growth and has a charge transfer that is higher than that of the conductive substrate.

7. The method of claim 6, in which forming the alloy (514) on the conductive substrate (522) comprises joining a support component (512) to the conductive substrate such that the alloy is formed between the conductive substrate and the support component.

8. The method of claim 7, in which joining the support substrate (512) to the conductive substrate (522) comprises laser welding or resistance welding.

9. The method of claim 7, further comprising removing the support component (512) to expose the alloy (514), in which removing the support substrate and dealloying the alloy comprises a chemical etching process.

10. The method of claim 6, in which forming an alloy (514) on the conductive substrate (522) comprises at least one of chemical vapor deposition, physical deposition, plating, and cladding.

11. The method of claim 7, in which the support component (512) comprises iron.

12. The method of claim 7, in which the support component (512) comprises an iron strip and a plating of a second metal over the iron strip.

13. The method of claim 12, in which the plating comprises at least one of:
copper, silver, iridium, and gold.

14. The method of claim 6, further comprising encapsulating conductors (555) and portions of the conductive substrate (522) in a flexible polymer (105, 575) prior to dealloying the alloy (514).

15. The method of claim 6, in which the alloy (514) is first formed on the conductive substrate (522), then the alloy is dealloyed to form a porous dealloyed electrode (100, 125, 400, 460, 570), then a signal wire (110, 555) is connected to the conductive substrate, then the signal wire and portions of the electrode are encapsulated using a molding process.

## Patentansprüche

1. Cochleaimplantatgerät mit:
einer Energiequelle;
mindestens einer Elektrode (100, 125, 400, 460, 570), welche mindestens zum Teil in einem flexiblen isolierenden Körper (105, 575) eingebettet ist und versehen ist mit:
einem elektrisch-leitenden Material (220, 522); und
einem nanoporösen entlegierten Material (514), welches auf einem Teil des elektrisch-leitenden Materials angeordnet ist und ausgebildet ist, um in elektrischem Kontakt mit dem biologischen Gewebe zu stehen, wobei das nanoporöse entlegierte Material eine Struktur (524) aufweist, die ausgebildet ist, um zellulärer Anhaftung und/oder faserigem Gewebewachstum zu widerstehen und einen Ladungstransfer aufzuweisen, der höher ist als derjenige des elektrisch-leitenden Materials; und
einem elektrischen Leiter (110, 555), der Strom zwischen der Energiequelle und der Elektrode leitet.

2. Gerät gemäß Anspruch 1, wobei das nanoporöse entlegierte Material (514) Iridium aufweist.

3. Gerät gemäß Anspruch 1, wobei nur Teile der mindestens einen Elektrode (100, 125, 400, 460, 570), welche dem biologischen Gewebe (115) ausgesetzt sind, mit dem nanoporösen entlegierten Material (514) bedeckt sind.

4. Gerät gemäß Anspruch 2, wobei das elektrisch-leitende Material (220, 522) Platin aufweist.

5. Gerät gemäß Anspruch 1, wobei ein exponierter geometrisch planarer Bereich des entlegierten Materials (514) weniger als 0,2 mm² ausmacht und die gesamte elektrochemische Oberfläche des entlegierten Materials mehr als das Zehnfache der geometrisch planaren Fläche beträgt.

6. Verfahren zum Bilden des Cochleaimplantatgeräts gemäß Anspruch 1, wobei mindestens eine implantierbare poröse entlegierte Cochleaelektrode (100, 125, 400, 460, 570) gebildet wird, indem:
ein Signaldraht (110, 555) an einem leitenden Substrat (220, 522) befestigt wird;
eine Legierung (514) auf dem leitenden Substrat gebildet wird; und
die Legierung entlegiert wird, um eine Oberflächenstruktur (524) zu bilden, die ausgebildet ist, um zellulärer Anhaftung und/oder faserigem Gewebewachstum zu widerstehen und einen Ladungstransfer aufweist, der größer ist als derjenige des leitenden Substrats.

7. Verfahren gemäß Anspruch 6, wobei beim Bilden der Legierung (514) auf dem leitenden Substrat (522) eine Trägerkomponente (512) mit dem leitenden Substrat so verbunden wird, dass die Legierung zwischen dem leitenden Substrat und der Trägerkomponente gebildet wird.

8. Verfahren gemäß Anspruch 7, wobei bei dem Verbinden des Trägersubstrats (512) mit dem leitenden Substrat (522) Laserschweißen oder Widerstandsschweißen verwendet wird.

9. Verfahren gemäß Anspruch 7, wobei ferner die Trägerkomponente (512) entfernt wird, um die Legierung (514) freizulegen, wobei das Entfernen des Trägersubstrats und das Entlegieren der Legierung einen chemischen Ätzprozess aufweisen.

10. Verfahren gemäß Anspruch 6, wobei bei dem Bilden der Legierung (514) auf dem leitenden Substrat (522) Dampfabscheidung, physikalische Abscheidung, Plattieren und/oder Ummanteln verwendet werden.

11. Verfahren gemäß Anspruch 7, wobei die Trägerkomponente (512) Eisen aufweist.

12. Verfahren gemäß Anspruch 7, wobei die Trägerkomponente (512) einen Eisenstreifen und eine Plattierung aus einem zweiten Metall über dem Eisenstreifen aufweist.

13. Verfahren gemäß Anspruch 12, wobei die Plattierung Kupfer, Silber, Iridium und/oder Gold aufweist.

14. Verfahren gemäß Anspruch 6, wobei ferner Leiter (555) und Teile des leitenden Substrats (522) vor dem Entlegieren der Legierung (514) in einem flexiblen Polymer (105, 575) eingekapselt werden.

15. Verfahren gemäß Anspruch 6, wobei die Legierung (514) zuerst auf dem leitenden Substrat (522) gebildet wird, dann die Legierung entlegiert wird, um eine poröse entlegierte Elektrode (100, 125, 400, 460, 570) zu bilden, dann ein Signaldraht (110, 555) mit dem leitenden Substrat verbunden wird, und dann der Signaldraht und Teile der Elektrode unter Verwendung eines Formungsprozesses umkapselt werden.

## Revendications

1. Dispositif d'implant cochléaire, comprenant :
une source d'énergie ;
au moins une électrode (100, 125, 400, 460, 570) partiellement noyée dans un corps isolant souple (105, 575), et comprenant :
un matériau conducteur de l'électricité (220, 522) ; et
un matériau désallié nanoporeux (514) disposé sur une portion du matériau conducteur de l'électricité, le matériau désallié nanoporeux étant configuré de façon à être en contact électrique avec le tissu biologique, le matériau désallié nanoporeux ayant une structure (524) qui est configurée pour résister à l'adhérence cellulaire et/ou à la croissance d'un tissu fibreux, et un transfert de charge qui est supérieur à celui du matériau conducteur de l'électricité ; et
un conducteur électrique (110, 555) qui conduit le courant entre la source d'énergie et l'électrode.

2. Dispositif selon la revendication 1, dans lequel le matériau désallié nanoporeux (514) comprend de l'iridium.

3. Dispositif selon la revendication 1, dans lequel seules sont recouvertes du matériau désallié nanoporeux (514) les portions de la ou des électrodes (100, 125, 400, 460, 570) qui sont exposées à un tissu biologique (115).

4. Dispositif selon la revendication 2, dans lequel le matériau conducteur de l'électricité (220, 522) comprend du platine.

5. Dispositif selon la revendication 1, dans lequel une aire plane géométrique exposée du matériau désallié (514) est inférieure à 0,2 millimètre carré, et l'aire superficielle électrochimique totale du matériau désallié est supérieure à 10 fois l'aire plane géométrique.

6. Procédé pour la formation du dispositif d'implant cochléaire selon la revendication 1, comprenant la formation d'au moins une électrode cochléaire désalliée poreuse implantable (100, 125, 400, 460, 570), comprenant :
la fixation d'un fil de transmission de signaux (110, 555) à un substrat conducteur (220, 522) ;
la formation d'un alliage (514) sur le substrat conducteur ; et
le désalliage de l'alliage pour former une structure superficielle (524) qui est configurée pour résister à l'adhérence cellulaire et/ou à la croissance d'un tissu fibreux et a un transfert de charge qui est supérieur à celui du substrat conducteur.

7. Procédé selon la revendication 6, dans lequel la formation de l'alliage (514) sur le substrat conducteur (522) comprend la réunion d'un composant support (512) au substrat conducteur de façon que l'alliage se forme entre le substrat conducteur et le composant support.

8. Procédé selon la revendication 7, dans lequel la réunion du substrat support (512) au substrat conducteur (522) comprend un soudage au laser ou un soudage par résistance.

9. Procédé selon la revendication 7, comprenant en outre l'enlèvement du composant support (512) pour exposer l'alliage (514), dans lequel l'enlèvement du substrat support et le désalliage de l'alliage comprennent une opération de gravure chimique.

10. Procédé selon la revendication 6, dans lequel la formation d'un alliage (514) sur le substrat conducteur (522) comprend au moins l'un d'un dépôt chimique en phase vapeur, d'un dépôt physique, d'une métallisation et d'un gainage.

11. Procédé selon la revendication 7, dans lequel le composant support (512) comprend du fer.

12. Procédé selon la revendication 7, dans lequel le composant support (512) comprend une bande de fer et l'application par métallisation d'un deuxième métal sur la bande de fer.

13. Procédé selon la revendication 12, dans lequel la métallisation comprend au moins l'un d'un cuivrage, d'un argentage, d'un iridiage et d'une dorure.

14. Procédé selon la revendication 6, qui comprend en outre l'encapsulation des conducteurs (555) et de portions du substrat conducteur (522) dans un polymère souple (105, 575) avant désalliage de l'alliage (514).

15. Procédé selon la revendication 6, dans lequel l'alliage (514) est d'abord formé sur le substrat conducteur (522), puis l'alliage est désallié pour former une électrode désalliée poreuse (100, 125, 400, 460, 570), puis un fil de transmission de signaux (110, 555) est connecté au substrat conducteur, puis le fil de transmission de signaux et des portions de l'électrode sont encapsulés par utilisation d'une opération de moulage.
